# EUROPEAN PATENT SPECIFICATION

(11) **EP 0 541 176 B1**
(45) Date of publication and mention of the grant of the patent: **31.01.1996**
(21) Application number: 92203389.9
(22) Date of filing: 04.11.1992
(51) Int. Cl.: C07C 209/60

(54) **Process for the preparation of secondary amines**
Verfahren zur Herstellung von sekundären Aminen
Procédé de préparation d'amines secondaires

(30) Priority: 07.11.1991 EP 91310314
(43) Date of publication of application: 12.05.1993
(73) Proprietor: SHELL INTERNATIONALE RESEARCH MAATSCHAPPIJ B.V., NL-2596 HR Den Haag (NL)
(72) Inventor: Van Zon, Arie, NL-1031 CM Amsterdam (NL); Van Asselen, Otto Leonardus Johannes, NL-1031 CM Amsterdam (NL); Drent, Eit, NL-1031 CM Amsterdam (NL); De Boer, Henricus Jacobus Robert, Gillingham, Kent ME7 3BD (GB); Van Es, Cees, Adisham, Kent CT3 3LG (GB)

(56) References cited:
- EP-A- 0 457 386
- FR-A- 2 476 642
- GB-A- 2 136 438
- US-A- 4 503 217
- US-A- 4 832 702

## Description

The present invention relates to a process for preparing secondary amines.

It is known to prepare secondary amines by reacting ammonia, or a primary amine with carbon monoxide, hydrogen and an olefinic compound. For example, US-A-4,503,217 discloses an aminomethylation process which comprises reacting a polymeric polyolefin, ammonia, primary or secondary amine and synthesis gas in the presence of a ruthenium-containing catalyst system and a single or two-phase solvent medium, heating the mixture to a temperature of at least 100°C and a pressure of at least 100 psi (689 kPa) until there is substantial formation of the desired polyamine and separating said polymeric polyamine product by a phase separation technique. The resulting polymeric products are useful as surfactants, surfactant precursors and wet strength agents for paper products.

US-A-4,832,702 discloses a fuel or lubricant composition which contains one or more polybutyl- or polyisobutylamines of the general formula I 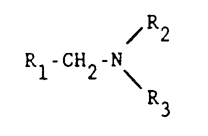
where R₁ is a polybutyl- or polyisobutyl radical derived from isobutene and up to 20% by weight of n-butene and R₂ and R₃ are identical or different and are each hydrogen, an aliphatic or aromatic hydrocarbon, a primary or secondary, aromatic or aliphatic aminoalkylene radical or polyaminoalkylene radical, a polyoxyalkylene radical or a hetaryl or heterocyclyl radical, or, together with the nitrogen atom to which they are bonded, form a ring in which further hetero atoms may be present. The compounds of general formula I are prepared via a two-step process:
i) hydroformylating an appropriate polybutene or polyisobutene with a rhodium or cobalt catalyst in the presence of CO and H₂ at from 80 to 200°C and CO/H₂ pressures of up to 600 bar (60,000 kPa), and
ii) subjecting the oxo product to a Mannich reaction or amination under hydrogenating conditions. The amination reaction is advantageously carried out at 80 to 200°C and under pressures up to 600 bar (60,000 kPa), preferably 80 to 300 bar (8,000 to 30,000 kPa).

US-A-4,526,936 discloses a method for forming polymeric products having a hydrocarbon backbone and a high degree of alkylene alkylamine pendant groups by contacting, in a liquid media, a polymer having unsaturated groups therein, a primary or secondary amine, carbon monoxide and hydrogen gas in the presence of a Group VIII metal containing compound. The formed product is useful for a variety of applications, for example, as a surfactant, a flocculating agent, softener and as a component in coating compositions.

EP-A-457,386 discloses a process for the preparation of an organic compound having at least one secondary amine group, which process comprises reacting ammonia or a primary amine with carbon monoxide, a hydrogen source and an olefinically unsaturated compound in the presence of a catalyst system comprising a source of cationic ruthenium, an aromatic N-heterocyclic ligand and a source of an anion other than a halide.

It has now surprisingly been found that secondary amines can be derived from polyolefins with a high conversion and selectivity using certain rhodium catalysts.

In accordance with the present invention there is provided a process for the preparation of a secondary amine which comprises reacting carbon monoxide, hydrogen, a polyolefin and a primary amine derivative having a terminal -NH₂ group and a terminal -NR¹R² group, wherein each of R¹ and R² independently represents a hydrogen atom or an alkyl group; in the presence of a suitable solvent and a catalyst system comprising a source of cationic rhodium; optionally a source of cationic ruthenium; an aromatic N-heterocyclic ligand and a source of an anion other than a halide.

In this specification, unless otherwise stated, an alkyl group may be linear or branched and preferably contains up to 20, more preferably up to 10, and especially up to 6, carbon atoms.

The polyolefin used in the process of the present invention may be partially hydrogenated, but it is important that there is some residual olefinic unsaturation in the polyolefin. The polyolefin has a number average molecular weight (Mₙ) preferably in the range of from 200 to 100,000, more preferably from 200 to 5,000, and especially from 500 to 2,500.

The polyolefins are polymers and oligomers derived from olefin monomers, preferably monomers containing from 2 to 30 carbon atoms, such as atactic polypropylene which may be conveniently prepared by a process analogous to the process of Comparative Example 1 of EP-A-268 214, or, more generally, to the process described in EP-A-69 951; partially hydrogenated polyisoprene which may be conveniently prepared by a process analogous to Example 1(a) of GB-A-1 575 507 with partial hydrogenation of the resulting polymer in accordance with Example 1(c); polyisobutylene; and polyalphaolefins, that is, partially hydrogenated oligomers, primarily trimers, tetramers and pentamers, of alphaolefin monomers containing generally from 6 to 12, more usually 8 to 12, carbon atoms, which may be prepared by a process as outlined in Hydrocarbon Processing, Feb. 1982, page 75 et seq; and, also, hydrocarbons prepared by a plasma process as described in EP-A-346,999.

Polyisobutylenes such as those sold by the British Petroleum Company under the Trade Marks "Ultravis" "Hyvis" and "Napvis" are particularly preferred for use in the process of the present invention.

The primary amine derivative may consist of carbon, hydrogen and nitrogen atoms or it may additionally contain oxygen and/or sulphur atoms. For example, the primary amine derivative may be a compound of the general formula: 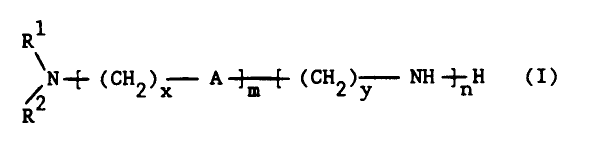
wherein each x is in the range 1 to 3, each y is in the range 1 to 3, each A independently represents -O- or -S-, m is 0, 1 or 2 and n is in the range 1 to 10, and wherein when m is 1, n is 1 and A is -O- or -S- or n is 2 and A is -S-, and when m is 2, n is 1; and R¹ and R² are as defined above.

In the primary amine derivative of formula I above, it is preferred that each of R¹ and R² independently represents a hydrogen atom or a C₁-C₆ alkyl, especially methyl, group. Particularly preferred primary amine derivatives of formula I are those wherein each of R¹ and R² independently represents a hydrogen atom or a methyl group, y is 2 or 3, m is 0 and n is in the range 1 to 6.

The primary amine derivative may be a single compound of formula I or mixture of two or more compounds of formula I.

Examples of primary amine derivatives of formula I above include 3-dimethylamino-1-propylamine, diethylene triamine, triethylene tetramine, tetraethylene pentamine and pentaethylene hexamine which may be obtained from the Aldrich Chemical Company Ltd., (UK); hexaethylene heptamine which may be obtained from Union Carbide Corporation (USA); H₂N-C₂H₄-S-C₂H₄-NH-C₂H₄-NH₂ (see US Patent No. 3,362,996); and H₂N-(C₂H₄-O)ₚ-C₂H₄-NH₂ (p=1,2) and H₂N-(C₂H₄-S)_{q}-C₂H₄-NH₂ (q=1,2) which are listed in Beilsteins Handbuch der Organischen Chemie (Springer-Verlag, Hamburg, Viertes Erganzungswerke).

Particularly preferred are 3-dimethylamino-1-propylamine, diethylene triamine, tetraethylene pentamine, pentaethylene hexamine, hexaethylene heptamine, and mixtures of tetraethylene pentamine, pentaethylene hexamine and hexaethylene heptamine.

The primary amine derivatives of formula I above may be prepared by the methods of H.R. Ing and R.H.F. Manske, J. Chem. Soc., 1926, p. 2348 et seq.; D.S. Tarbell, N. Shakespeare, C.J. Claus and J.R. Bunnett, J. Am. Chem. Soc., 1946, 68, p. 1217 et seq.; R.C. O'Gee and H.M. Woodburn, J. Am. Chem. Soc., 1951, 73, p. 1370 et seq.; A.F. McKay, D.L. Garmaise and A. Halsaz, Can. J. Chem., 1956, 34, p. 1567 et seq; Kirk-Othmer Encyclopedia of Chemical Technology, Volume 7 (Wiley Interscience, New York, 3rd edition, 1979) pp 580-602; US Patent No. 2,318,729; S. Gabriel, Berichte, 1891, 24, pp 1110-1121; F.P.J. Dwyer and F. Lions, J. Am. Chem. Soc., 1950, 72, pp 1545-1550; F.P.J. Dwyer, N.S. Gill, E.C. Gyarfas and F. Lions, J. Am. Chem. Soc., 1953, 75, pp 1526-1528; US Patent No. 3,362,996; or by processes analogous thereto.

The process according to the invention is conveniently carried out at a temperature in the range of from 80 to 250°C, preferably from 100 to 200°C, and especially from 110 to 180°C, advantageously 160 to 170°C.

The present process is conveniently carried out at a total pressure as determined at ambient temperature (20°C) of from 200 to 25,000 kPa, preferably from 2,000 to 20,000 kPa, and especially from 2,000 to 6,000 kPa. It will be appreciated, however, that when the process is operated batchwise at a constant temperature, the pressure will fall during the course of reaction.

A source of cationic rhodium or, if present, ruthenium may be any material comprising rhodium or ruthenium which is capable of yielding cationic rhodium or ruthenium species. Examples of suitable sources include compounds of rhodium or ruthenium such as oxides; salts such as nitrates, sulphates, sulphonates (e.g. trifluoromethanesulphonates or p-toluenesulphonates), borates (e.g. tetrafluoroborates) phosphates, phosphonates (e.g. benzenephosphonates), carboxylates (e.g. acetates, propionates or butyrates), and acetylacetonates (e.g. Rh(CO)₂(acac) or Ru(acac)₃ where acac denotes an acetylacetonate anion); carbonyls (e.g. triruthenium dodecacarbonyl) and hydrides (e.g. HRh(CO)(Ph₃P)₃, H₂Ru₄(CO)₁₃, or H₄Ru₄(CO)₁₂).

The aromatic N-heterocyclic ligand used in the process of the present invention may be monodentate or multidentate containing a plurality of coordinative N-ring atoms. Preferably it is a bidentate ligand containing two coordinative nitrogen atoms. It will be appreciated that the ligand should be inert. The ligand may comprise one or more, e.g. up to 6, preferably up to 4, substituents on the N-heterocyclic ring or rings, which substituents may be linked together to form condensed ring systems such as phenanthroline or quinoline. Examples of substituents include carboxyl, alkyl, alkoxy, alkylthio, alkoxycarbonyl, alkylamino, dialkylamino, cycloalkyl and aryl groups. Any alkyl moiety in such substituents may be linear or branched and preferably contains up to 6, more preferably up to 4, carbon atoms. A cycloalkyl substituent group preferably contains from 3 to 8, more preferably 3 to 6, carbon atoms. An aryl substituent group may be any aromatic hydrocarbon group, especially a phenyl or naphthyl group.

The expression "aromatic N-heterocyclic ligand" as used herein indicates an organic ring compound containing at least one imino N-ring atom and a plurality of double bonds in the ring structure, such that the electron density is at least partially delocalised. The ring may be five-, six- or seven-membered, and may contain, besides carbon and nitrogen atoms, other hetero atoms such as oxygen or sulphur. Two aromatic N-heterocyclic rings may be linked together to form a bidentate ligand such as in 2,2'-bipyridine or 1,10-phenanthroline.

Preferably the aromatic N-heterocyclic ligand is an optionally substituted pyridine, quinoline, 2,2'-bipyridine or 1,10-phenanthroline.

Examples of aromatic N-heterocyclic ligands are pyridines, e.g. pyridine, 3-methylpyridine, 4-methylpyridine, 2,6-dimethylpyridine, 4-ethylpyridine, 2-methoxypyridine, 2-dimethylaminomethylpyridine, 2-pyridinecarboxylic acid; quinolines, e.g. quinoline or 2-methylquinoline; 2,2'-bipyridines, e.g. 2,2'-bipyridine, 4,4'-di-t-butyl-2,2'-bipyridine, 4,4'-diphenyl-2,2'-bipyridine, 4,4'-dicarboxyl-2,2'-bipyridine, 3,3'-dimethyl-2,2'-bipyridine, 4,4'-dimethyl-2,2'-bipyridine, 5,5'-dimethyl-2,2'-bipyridine or 6,6'-dimethyl-2,2'-bipyridine; 1,10-phenanthrolines, e.g. 1,10-phenanthroline, 4,7-dimethyl-1,10-phenanthroline, 3,4,7,8-tetramethyl-1,10-phenanthroline, 4,7-diphenyl-1,10-phenanthroline, or 3,4,5,6,7,8-hexamethyl-1,10-phenanthroline. Further suitable ligands include 2-(2-pyridyl)benzimidazole, 3-(2-pyridyl)-5,6-diphenyl-1,2,4-triazine, the monosodium salt of 3-(2-pyridyl)-5,6-diphenyl-1,2,4-triazine-p,p'-disulphonic acid and the disodium salt of 4,4'-dicarboxyl-2,2'-bipyridine.

Particularly preferred ligands are 2,2'-bipyridine, 4,4'-diphenyl-2,2'-bipyridine, 4,4'-dimethyl-2,2'-bipyridine, 4,4'-dicarboxyl-2,2'-bipyridine, 1,10-phenanthroline, 3,4,7,8-tetramethyl-1,10-phenanthroline and 4,7-diphenyl-1,10-phenanthroline.

The source of an anion other than a halide may be a salt or an acid. It may also be a metal complex, for example a rhodium or ruthenium complex. The anion is preferably a nitrate, sulphate, sulphonate, borate, phosphate, phosphonate, carboxylate or acetylacetonate anion. Preferably the source of anion is a complex or salt of rhodium or ruthenium, or an acid.

The number of moles of aromatic N-heterocyclic ligand per gram atom of rhodium is preferably in the range of from 0.5 to 1000, more preferably from 0.75 to 50, and especially from 1 to 10.

The number of moles of anion per gram atom of rhodium is preferably at least 0.5, more preferably in the range of from 1 to 100, and especially from 2 to 50.

The number of gram atoms of rhodium used per mole of polyolefin is not critical. It is preferably in the range of from 10⁻⁶ to 10² gram atoms rhodium per mole of polyolefin.

The ratio of the number of gram atoms of rhodium to ruthenium preferably ranges from 100:1 to 1:100, more preferably from 100:1 to 1:10, and especially from 10:1 to 1:1.

The CO to H₂ ratio preferably ranges from 1:10 to 10:1, more preferably from 1:5 to 5:1.

The process of the invention is carried out in the presence of a solvent. Typical examples of solvents are ethers such as diethyleneglycol dimethylether (diglyme), 1,4-dioxane or tetrahydrofuran; amides such as dimethylformamide or dimethylacetamide; alcohols such as hexanol or tetraethylene glycol; esters such as ethyl acetate; and hydrocarbons such as hexane, cyclohexane, toluene and the xylenes.

The secondary amines prepared according to the process of the present invention may be used as additives in lubricating oil compositions. The amount of secondary amine used is preferably from 0.1 to 10%w, especially from 0.5 to 5%w, based on total composition.

Suitable lubricating oils are natural, mineral or synthetic lubricating oils.

Natural lubricating oils include animal and vegetable oils, such as castor oil. Mineral oils comprise the lubricating oil fractions derived from crude oils, coal or shale, which fractions may have been subjected to certain treatments such as clay-acid, solvent or hydrogenation treatments. Synthetic lubricating oils include synthetic polymers of hydrocarbons, modified alkylene oxide polymers, and ester lubricants, which are known in the art. These lubricating oils are preferably crankcase lubricating oils for spark-ignition and compression-ignition engines, but include also hydraulic lubricants, metal-working fluids and automatic transmission fluids.

The lubricating oil compositions may contain various other additives known in the art, such as viscosity index improvers, e.g. linear or star-shaped polymers of a diene such as isoprene or butadiene, or a copolymer of such a diene with optionally substituted styrene. These copolymers are suitably block copolymers and are preferably hydrogenated to such an extent as to saturate most of the olefinic unsaturation. Other suitable additives include dispersant V.I. improvers such as those based on block copolymers, or polymethacrylates, extreme pressure/anti-wear additives such as zinc or sodium dithiophosphates, ashless dispersants such as polyolefin-substituted succinimides, e.g. those described in GB-A-2 231 873, anti-oxidants, friction modifiers or metal-containing detergents such as phenates, sulphonates, alkylsalicylates or naphthenates, all of which detergents may be overbased.

The secondary amines prepared according to the process of the present invention may also be used as additives in fuel compositions. The amount of secondary amine used is preferably from 0.1 to 10%w, especially from 0.5 to 5%w, based on total composition.

Suitable fuels include hydrocarbon base fuels boiling esentially in the gasoline boiling range from 30 to 230°C. These base fuels may comprise mixtures of saturated, olefinic and aromatic hydrocarbons. They can be derived from straight-run gasoline, synthetically produced aromatic hydrocarbon mixtures, thermally or catalytically cracked hydrocarbon feedstocks, hydrocracked petroleum fractions or catalytically reformed hydrocarbons.

The lubricating oil or fuel compositions may conveniently be prepared by blending an additives concentrate into the lubricating base oil or fuel. Such a concentrate generally comprises an inert carrier fluid and one or more additives in a concentrated form. In the preparation of the lubricating oil compositions, the concentrate conveniently contains a lubricating oil as inert carrier fluid, whilst in the preparation of the fuel compositions, the concentrate conveniently contains a fuel as inert carrier fluid. Typically, the secondary amine will be present in the additive concentrate in an amount from 10 to 80%w, based on the total concentrate.

The invention will be further understood from the following illustrative examples, in which the following polyolefins were used: the polyisobutylenes sold by The British Petroleum Company under the Trade Marks "Ultravis 10", "Ultravis 30", "Hyvis 10", "Hyvis 30", "Hyvis 150" and "Napvis 10" having respectively a number average molecular weight (Mₙ) of 970, 1300, 1025, 1320, 2400 and 971, the Mₙ values being determined by gel permeation chromatography ("Ultravis 10", "Ultravis 30", "Hyvis 10" and "Napvis 10") or by quantitative reaction with ozone ("Hyvis 30" and "Hyvis 150"); partially hydrogenated polyisoprene (95%) having Mₙ of 90,000 as determined by gel permeation chromatography, which was prepared by a process analogous to Example 1(a) of GB-A-1 575 507 with partial hydrogenation of the resulting polymer in accordance with Example 1(c); a plasma produced oil having a viscosity at 100°C of 26 cSt (mm²/S) and an unsaturation level of 4000 mg bromine/100g prepared according to the process of EP-A-346,999; a C₁₀H₂₀-trimer (a polyalphaolefin, ex. Neste Chemicals); and atactic polypropylenes having Mₙ of 1150, 1250, 1700 and 1780, determined by quantitative reaction with ozone, which were prepared using as catalyst a bis(cyclopentadienyl) zirconium dichloride and a methyl aluminoxane in a manner analogous to the process of Comparative Example 1 of EP-A-268 214, or, more generally, to the process described in EP-A-69 951.

### Example 1

100mmol "Hyvis 10" polyisobutylene was introduced into a 250ml "Hastelloy C" (Trade Mark) metal autoclave together with 150mmol 3-dimethylamino-1-propylamine, 100ml diethyleneglycol dimethylether and, as catalyst, 0.2mmol Ru(acac)₃/0.4mmol Rh(CO)₂(acac)/2.4mmol 2,2'-bipyridine (acac denotes an acetylacetonate anion). The autoclave was then pressurised with a mixture of carbon monoxide and hydrogen (P_{CO}=3000 kPa, P_{Hydrogen}= 2500 kPa as determined at ambient temperature (20°C)) and the contents of the autoclave heated, with stirring, at 170°C for a period of 24 hours. After cooling, a product-containing phase was separated from a solvent-containing phase. The former was then added to n-heptane (product/n-heptane ratio of 1/5) and the resulting mixture was washed seven times with 25ml portions of water and then dried over anhydrous magnesium sulphate. Evaporation of the n-heptane under reduced pressure yielded the reaction product.

### Examples 2 to 76

By processes similar to that described in Example 1, further secondary amines were prepared. Details of the reagents used and the reaction conditions employed in each of the Examples are shown in Tables I and IA below in which the following abbreviations are used:-
- diglyme :: diethyleneglycol dimethylether
- DAP :: 3-dimethylamino-1-propylamine
- DMF :: dimethylformamide
- THF :: tetrahydrofuran
- DETA :: diethylene triamine
- APO :: atactic polypropylene
- TEPA :: tetraethylene pentamine
- PEHA :: pentaethylene hexamine
- mixed PEHA :: tetraethylene pentamine/pentaethylene hexamine/hexaethylene heptamine (1/2/1)
- HEPA :: hexaethylene heptamine
Active matter, basic-N, secondary-N, tertiary-N, total-N and total-O analysis data for the reaction products of Examples 1 to 76 are given, where known, in Table II below.

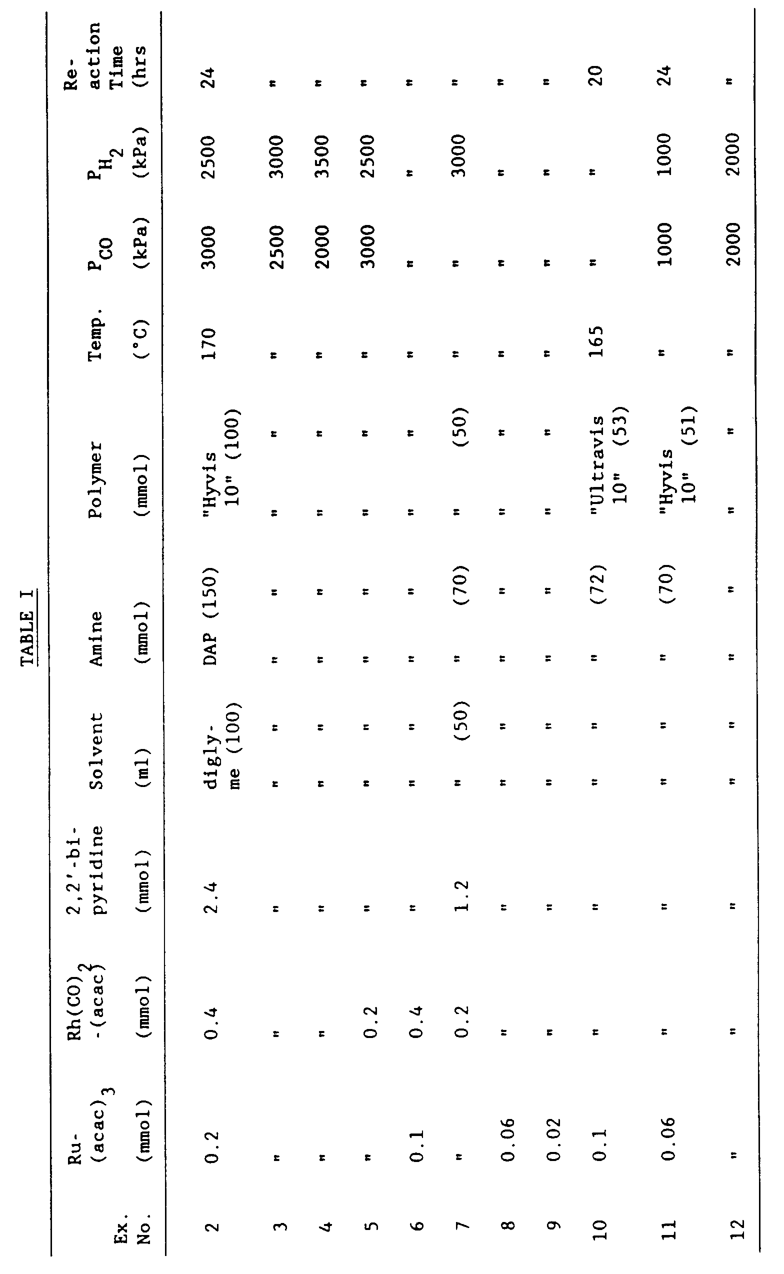

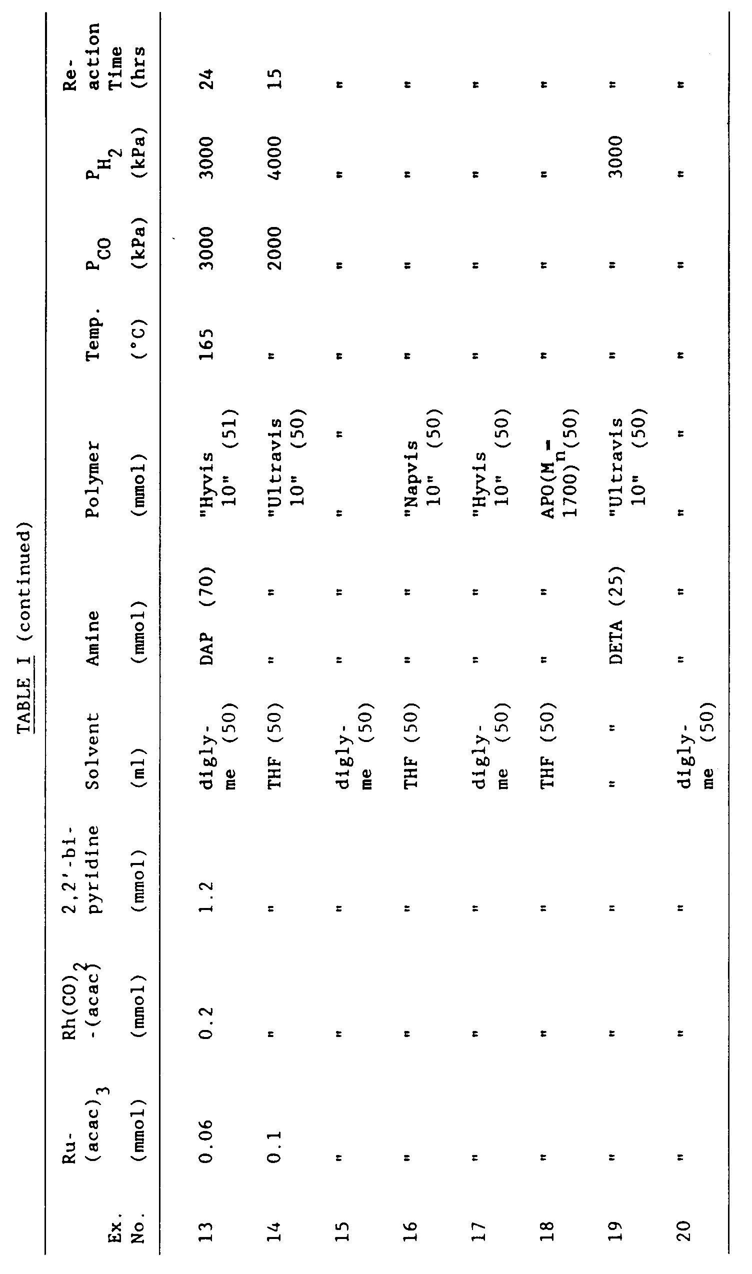

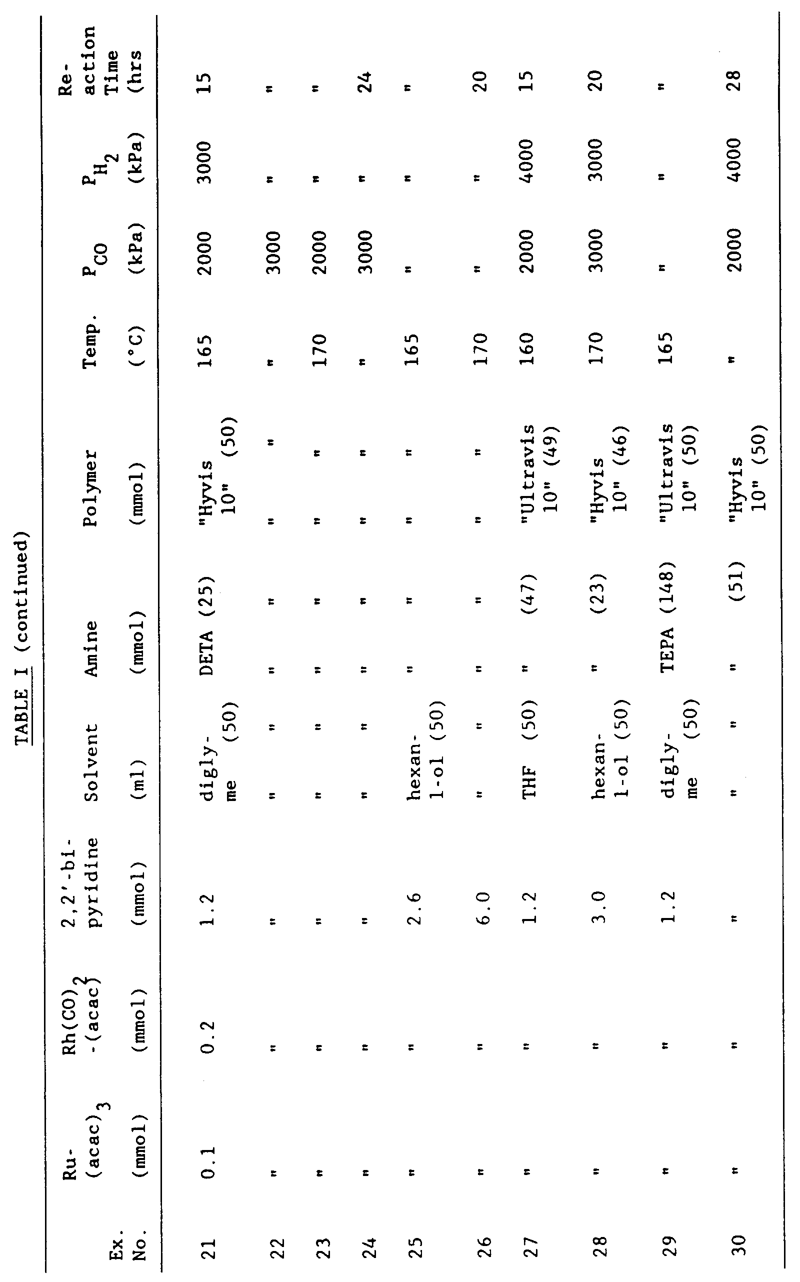

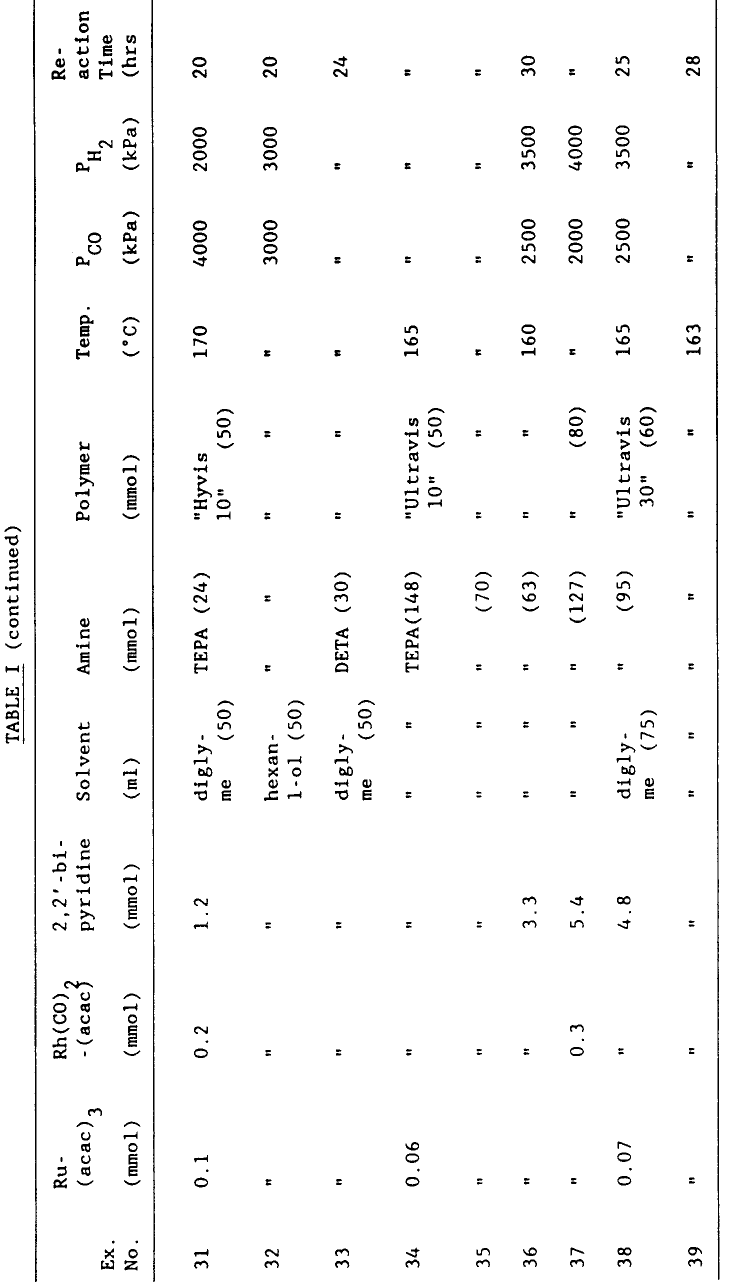

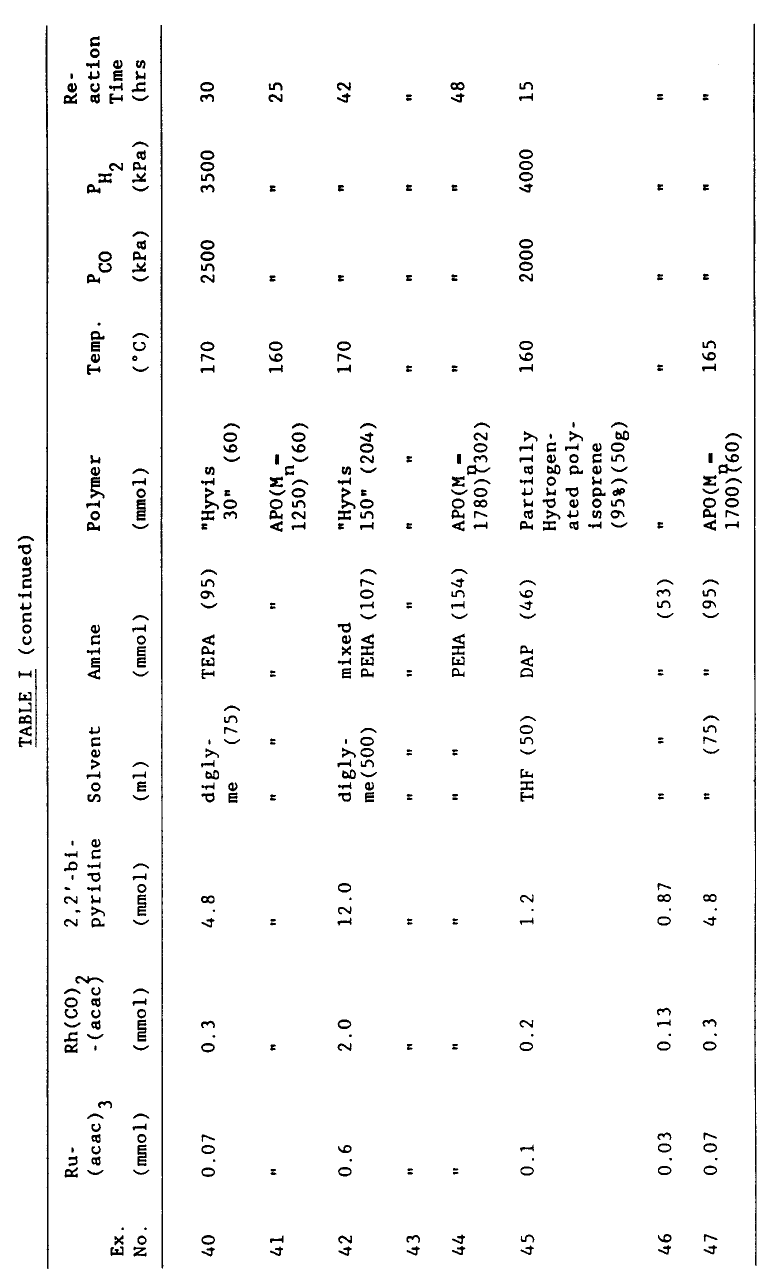

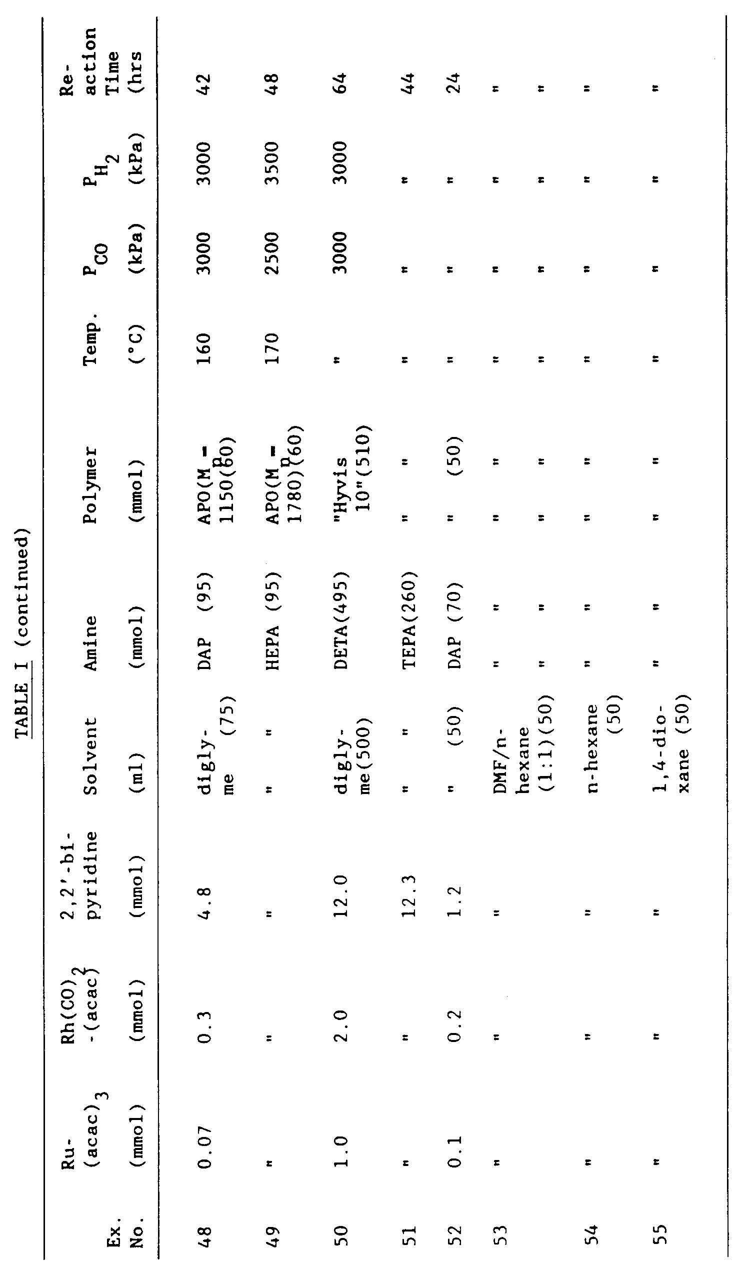

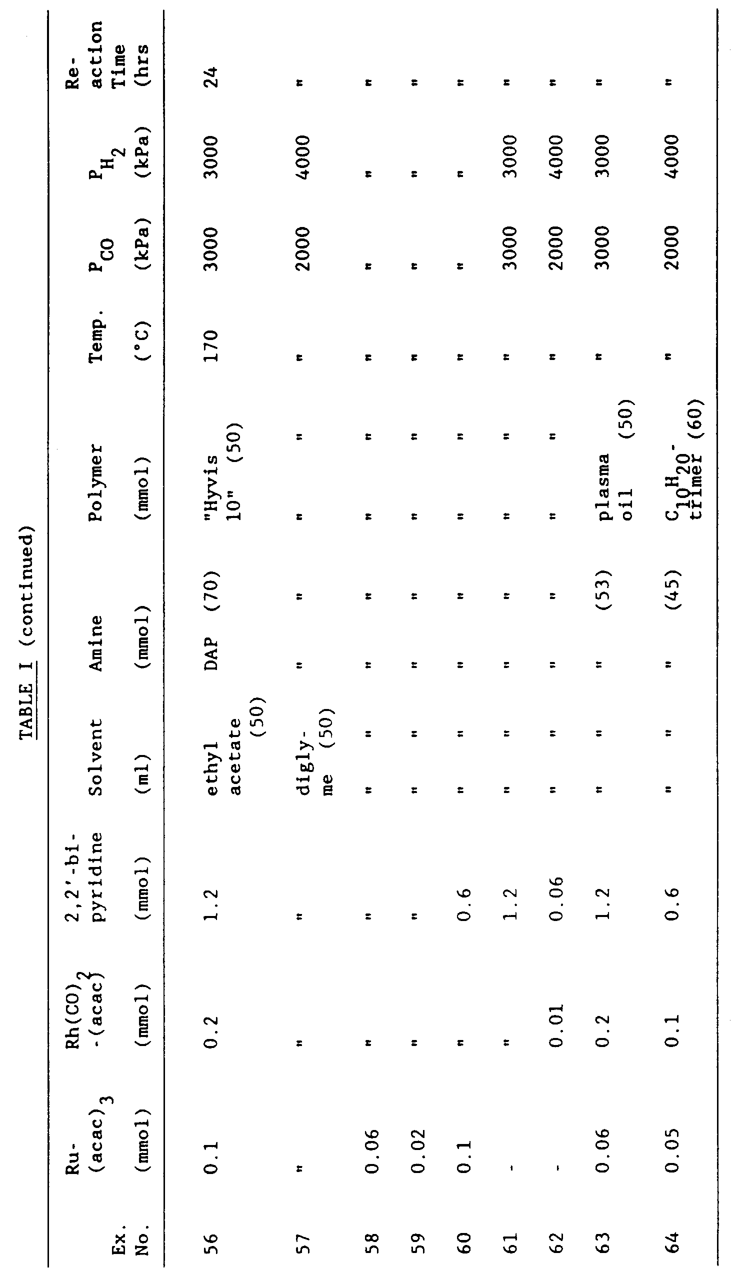

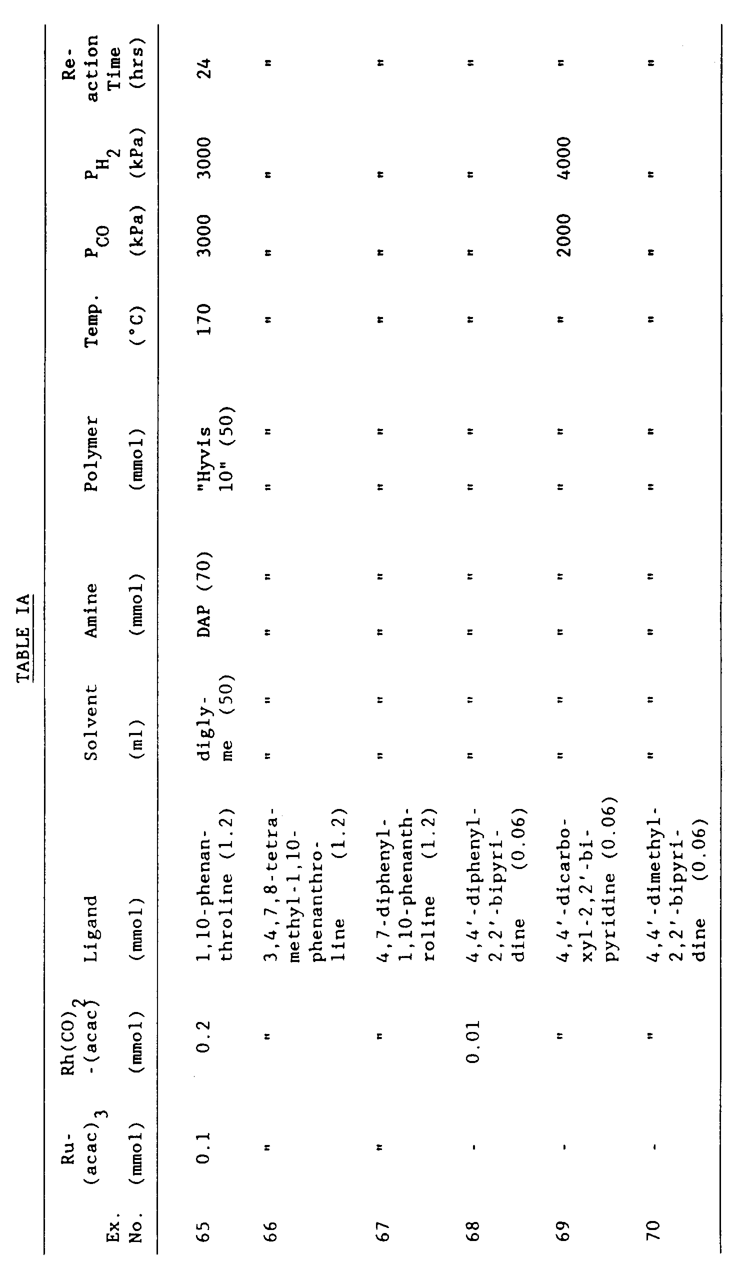

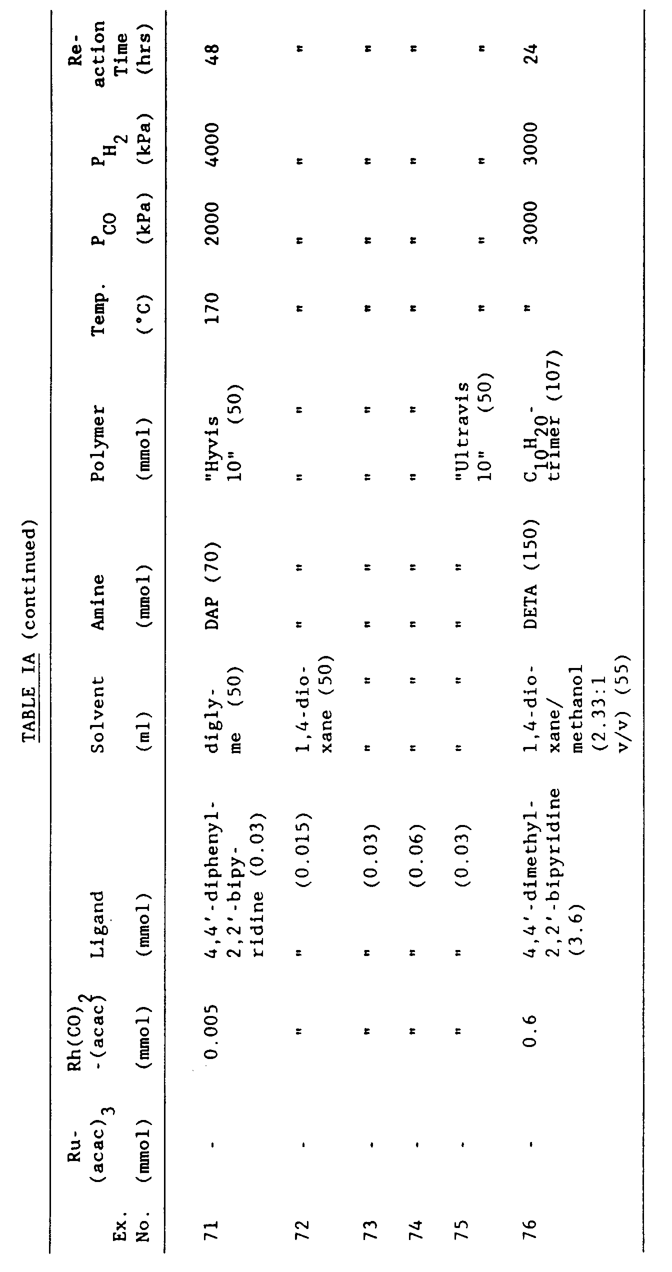

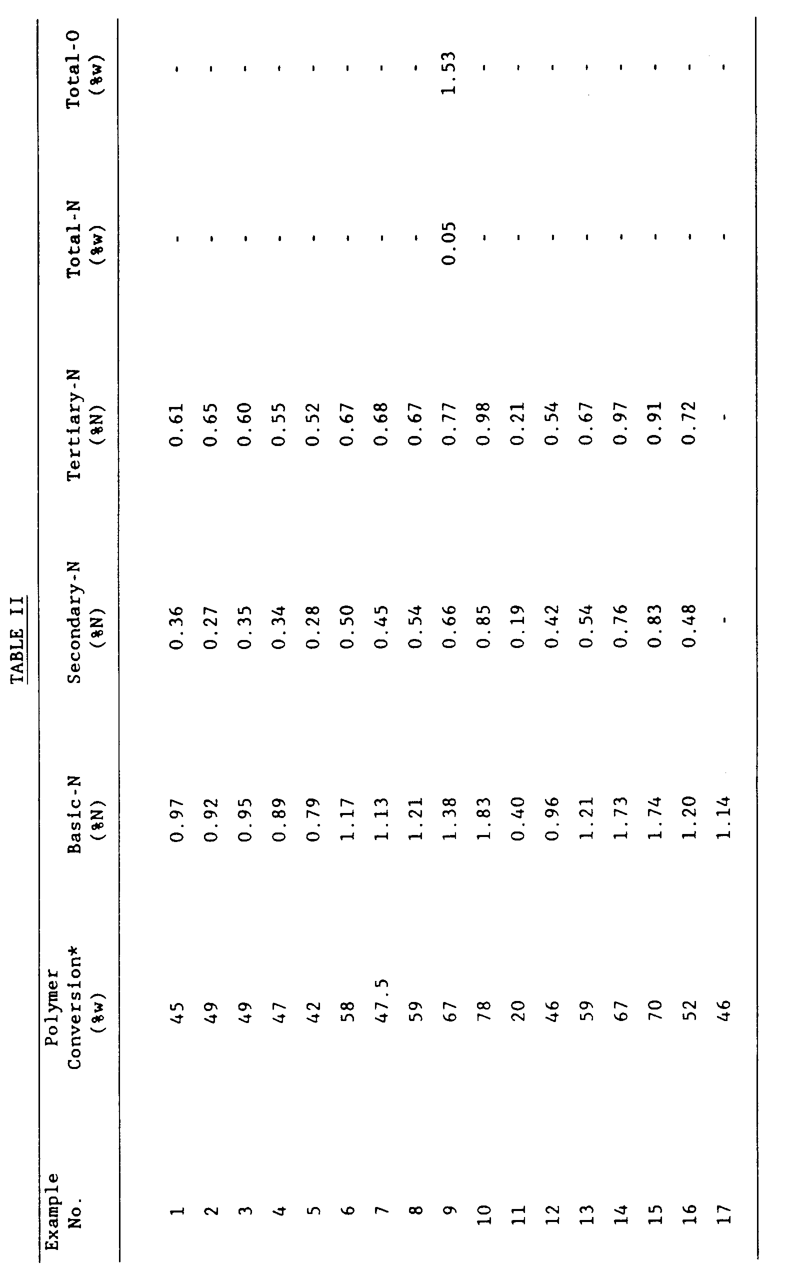

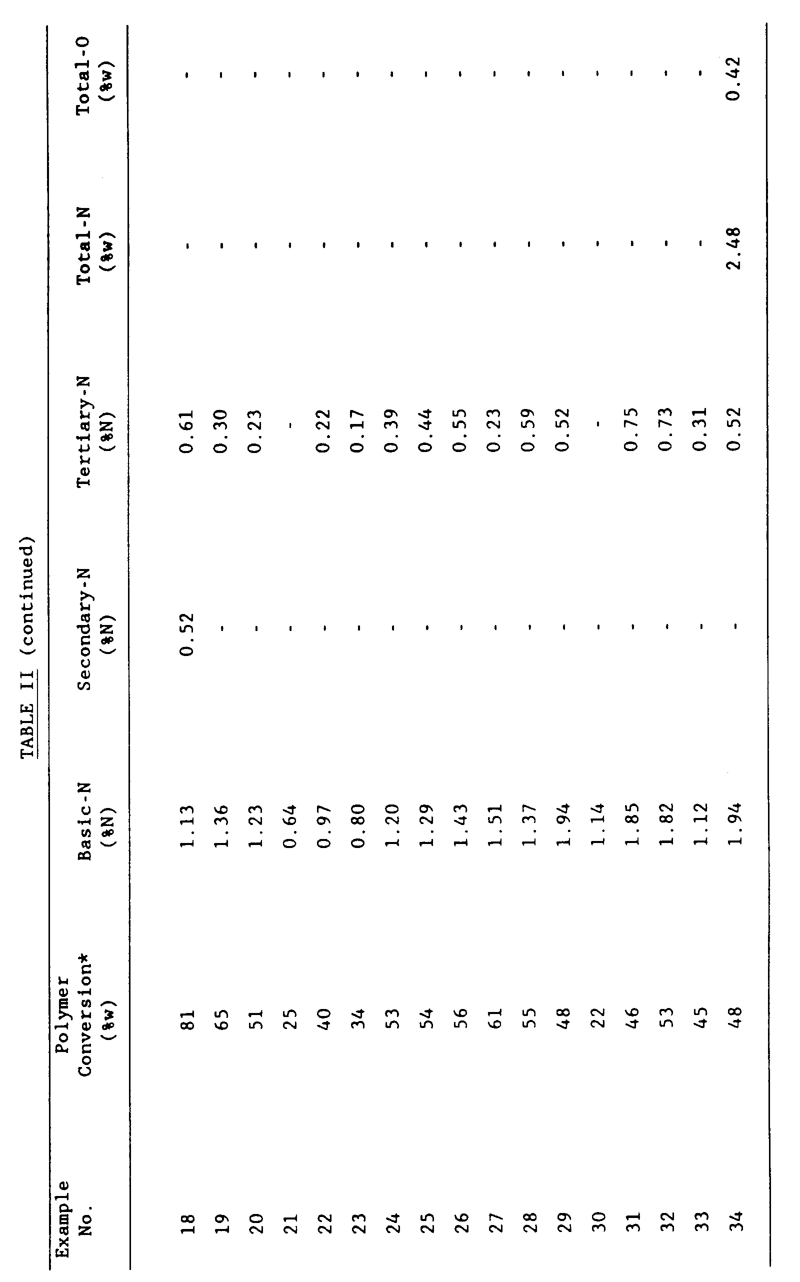

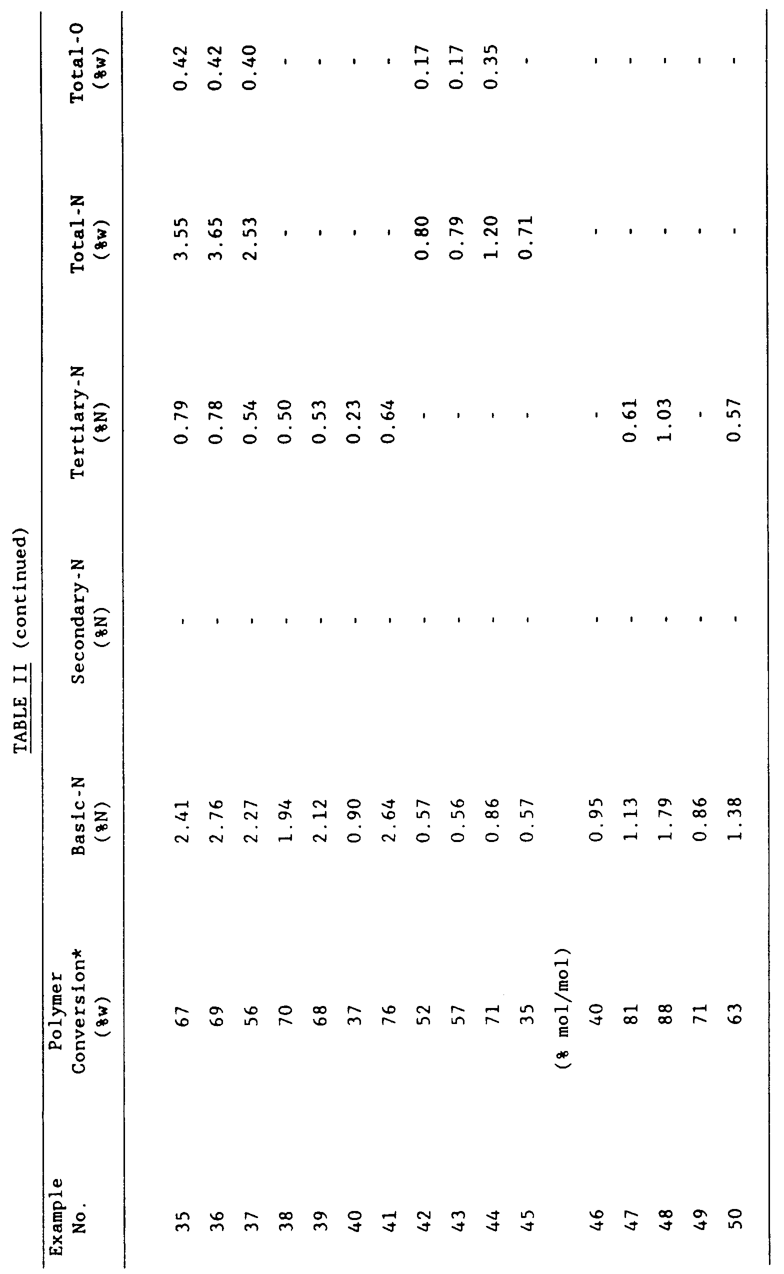

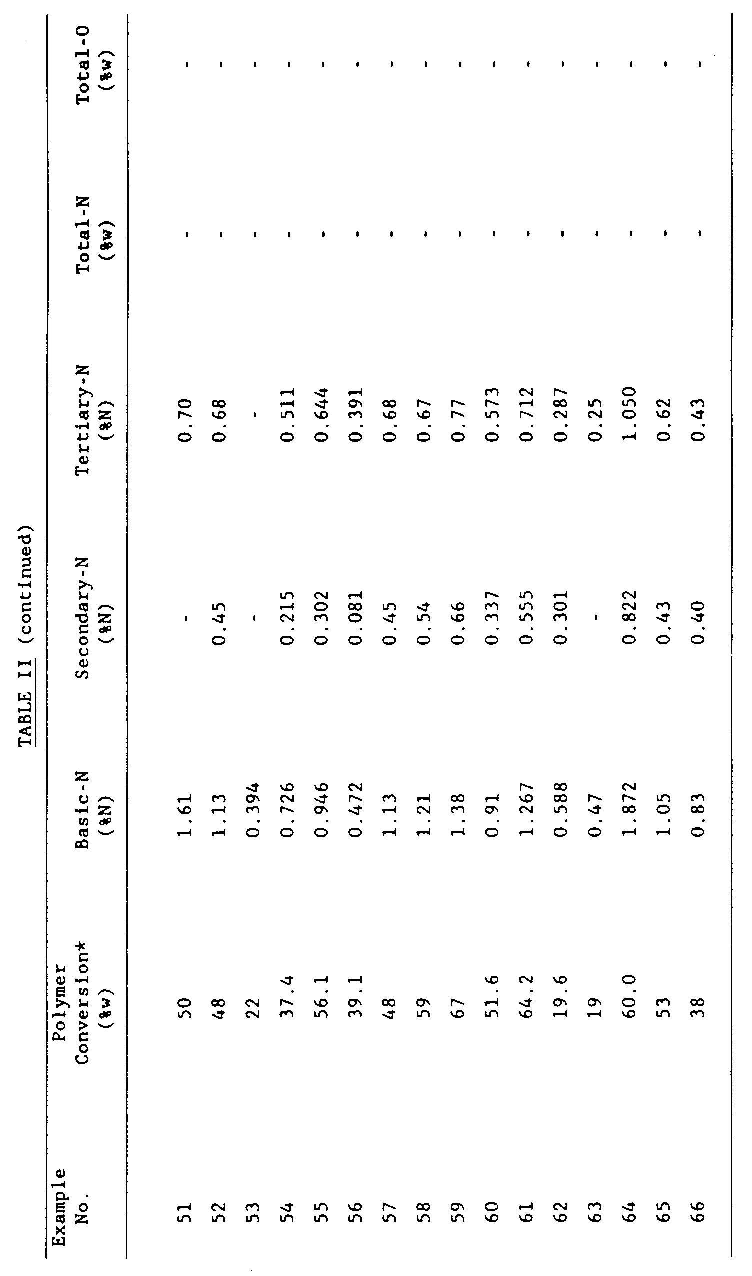

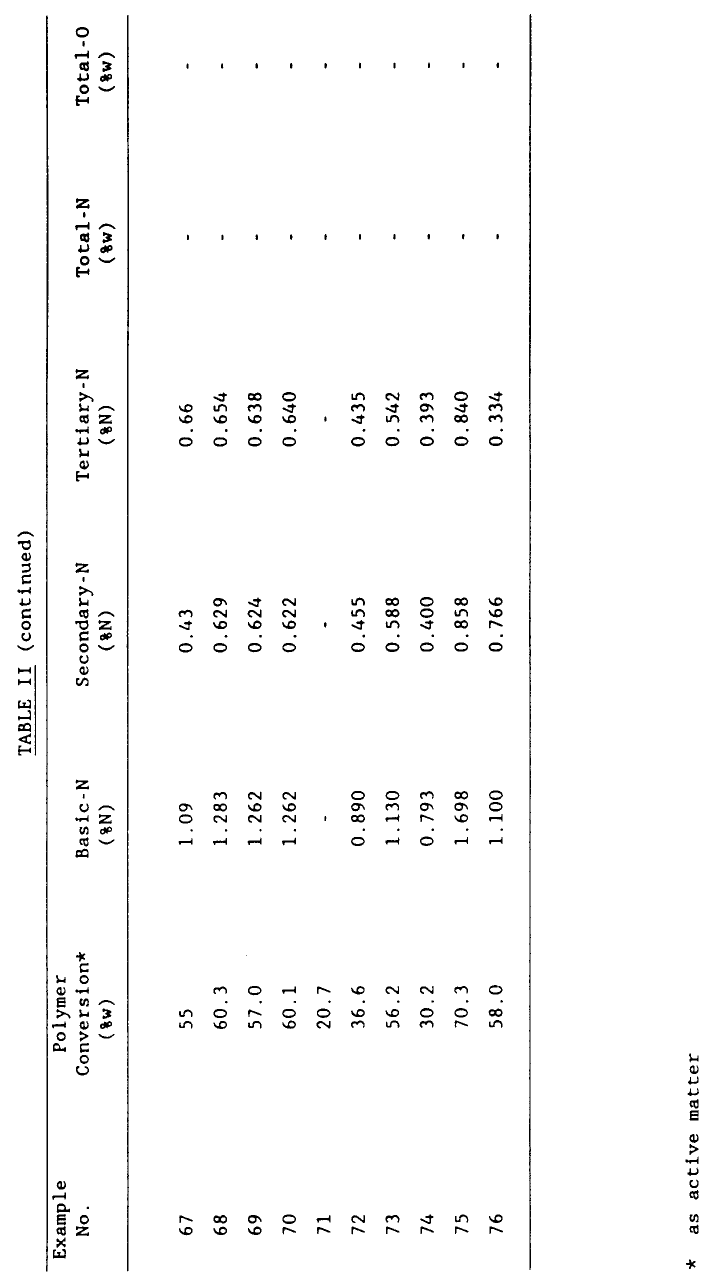

### Example 77

### Carbon Black Dispersancy Test (CBDT) (British Rail publication BR 669 : 1984)

The products of Examples 19, 20, 24 to 28, 32 and 42 to 44 were each diluted to an active matter content of 50%w by addition of "HVI 60" base oil (a bright and clear high viscosity index base oil having viscosity at 100°C 4.4 to 4.9 mm²/s (ASTM D 2270)). Each of the resulting concentrates was then added to samples of a SAE 15W40 Middle East lubricating oil containing a commercial package of a zinc dialkyldithiophosphate, an overbased calcium alkyl salicylate and VI improver, to give an oil containing the product at a concentration of 1%w active matter. 3%w of carbon black was then added to each oil and (percentage) increase in kinematic viscosity at 60^{o}C was determined, using an Ubbelohde viscometer. A low result indicates good performance.

The results of this test are given in Table III following:

**TABLE III**

| Product of Example No. | CBDT (%) |
|---|---|
| 19 | 33 |
| 20 | 33 |
| 24 | 58 |
| 25 | 62 |
| 26 | 54 |
| 27 | 38 |
| 28 | 63 |
| 32 | 41 |
| 42 | 58 |
| 43 | 58 |
| 44 | 52 |

### Example 78

### Engine Tests

Each of the products of Examples 50 and 51 was blended in an amount giving 2.5%w active matter in a base oil containing some 10.82%w of an additive package comprising overbased salicylate detergent, VI (viscosity index) improver, zinc-based anti-wear additive and polymethacrylate pour-point depressant. The resulting oils were evaluated according to sequence VE ASTM (as described in "Sequence VE test procedure", 7th draft dated 19th May 1988; ASTM Monitoring Centre, 4400 5th Avenue, Pittsburgh, USA).

For the purposes of comparison, similar evaluations were carried out on commercially available polyisobutylene-derived bis-succinimide ashless dispersant "SAP 220", at the same active matter concentration. Results of the tests are given in Table IV following.

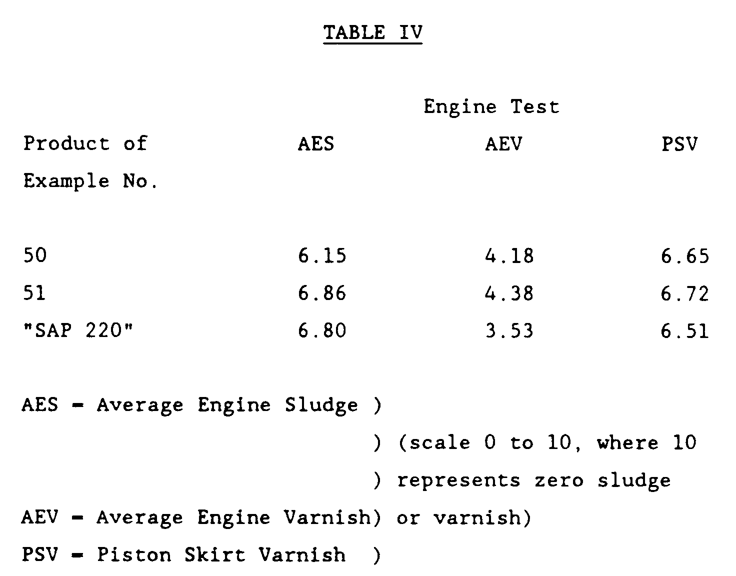

## Claims

1. A process for the preparation of a secondary amine which comprises reacting carbon monoxide, hydrogen, a polyolefin and a primary amine derivative having a terminal -NH₂ group and a terminal -NR¹R² group, wherein each of R¹ and R² independently represents a hydrogen atom or an alkyl group; in the presence of a suitable solvent and a catalyst system comprising a source of cationic rhodium; optionally a source of cationic ruthenium; an aromatic N-heterocyclic ligand and a source of an anion other than a halide.

2. A process according to claim 1, wherein the polyolefin has a number average molecular weight (Mₙ) in the range of from 200 to 100,000.

3. A process according to claim 1 or claim 2, wherein the polyolefin is polyisobutylene.

4. A process according to any one of claims 1 to 3, wherein the primary amine derivative is a compound of the general formula: 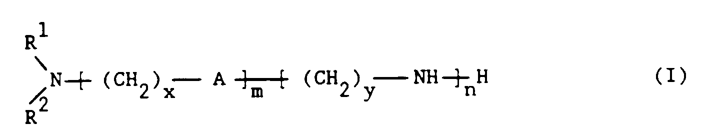 wherein each x is in the range 1 to 3, each y is in the range 1 to 3, each A independently represents -O- or -S-, m is 0, 1 or 2 and n is in the range 1 to 10, and wherein when m is 1, n is 1 and A is -O- or -S- or n is 2 and A is -S-, and when m is 2, n is 1; and R¹ and R² are as defined in claim 1.

5. A process according to claim 4, wherein in formula I each of R¹ and R² independently represents a hydrogen atom or a methyl group, y is 2 or 3, m is 0 and n is in the range 1 to 6.

6. A process according to any one of the preceding claims which is carried out at a temperature in the range of from 160 to 170°C.

7. A process according to any one of the preceding claims which is carried out at a total pressure in the range of from 2,000 to 6,000 kPa.

8. A process according to any one of the preceding claims, wherein the source of cationic rhodium or ruthenium is an oxide, salt, hydride or carbonyl.

9. A process according to any one of the preceding claims, wherein the aromatic N-heterocyclic ligand is an optionally substituted pyridine, quinoline, 2,2'-bipyridine, or 1,10-phenanthroline.

10. A process according to any one of the preceding claims, wherein the anion is a nitrate, sulphate, sulphonate, borate, phosphate, phosphonate, carboxylate or acetylacetonate anion.

## Patentansprüche

1. Verfahren zur Herstellung eines sekundären Amins, das ein Umsetzen von Kohlenmonoxid, Wasserstoff, einem Polyolefin und einem primären Aminderivat mit einer endständigen -NH₂-Gruppe und einer endständigen -NR¹R²-Gruppe, worin jeder der Reste R¹ und R² unabhängig ein Wasserstoffatom oder eine Alkylgruppe darstellt; in Anwesenheit eines geeigneten Lösungsmittels und eines Katalysatorsystems umfaßt, das eine Quelle für kationisches Rhodium; gewünschtenfalls eine Quelle für kationisches Ruthenium; einen aromatischen N-heterocyclischen Liganden und eine Quelle für ein von einem Halogenid unterschiedliches Anion umfaßt.

2. Verfahren nach Anspruch 1, worin das Polyolefin ein zahlenmittleres Molekulargewicht (Mₙ) im Bereich von 200 bis 100.000 aufweist.

3. Verfahren nach Anspruch 1 oder 2, worin das Polyolefin Polyisobutylen ist.

4. Verfahren nach einem der Ansprüche 1 bis 3, worin das primäre Aminderivat eine Verbindung der allgemeinen Formel: 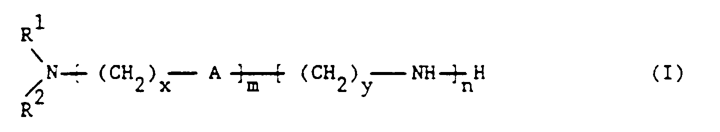 ist, worin jedes x im Bereich 1 bis 3 liegt, jedes y im Bereich 1 bis 3 liegt, jedes A unabhängig für -O- oder -S- steht, m den Wert 0, 1 oder 2 aufweist und n im Bereich 1 bis 10 liegt, und worin dann, wenn m für 1 steht, n den Wert 1 hat und A für -O- oder -S- steht, oder n den Wert 2 hat und A für -S- steht, und dann, wenn m 2 bedeutet, n den Wert 1 hat; und R¹ und R² wie in Anspruch 1 definiert sind.

5. Verfahren nach Anspruch 4, worin in Formel I jeder der Reste R¹ und R² unabhängig für ein Wasserstoffatom oder eine Methylgruppe steht, y den Wert 2 oder 3 hat, m den Wert 0 aufweist und n im Bereich von 1 bis 6 liegt.

6. Verfahren nach einem der vorstehenden Ansprüche, das bei einer Temperatur im Bereich von 160 bis 170°C ausgeführt wird.

7. Verfahren nach einem der vorstehenden Ansprüche, das bei einem Gesamtdruck im Bereich von 2.000 bis 6.000 kPa ausgeführt wird.

8. Verfahren nach einem der vorstehenden Ansprüche, worin die Quelle für kationisches Rhodium oder Ruthenium ein Oxid, Salz, Hydrid oder Carbonyl ist.

9. Verfahren nach einem der vorstehenden Ansprüche, worin der aromatische N-heterocyclische Ligand ein gegebenenfalls substituiertes Pyridin, Chinolin, 2,2'-Bipyridin oder 1,10-Phenanthrolin ist.

10. Verfahren nach einem der vorstehenden Ansprüche, worin das Anion ein Nitrat-, Sulfat-, Sulfonat-, Borat-, Phosphat-, Phosphonat-, Carboxylat- oder Acetylacetonatanion ist.

## Revendications

1. Procédé de préparation d'une amine secondaire, caractérisé en ce que l'on fait réagir du monoxyde de carbone, de l'hydrogène, une polyoléfine et un dérivé d'amine primaire possédant un radical -NH₂ terminal et un radical -NR¹R² terminal, où chacun des symboles R¹ et R² représente indépendamment un atome d'hydrogène ou un radical alkyle, en présence d'un solvant approprié et d'un système catalytique comprenant une source de rhodium cationique, éventuellement une source de ruthénium cationique, un ligand N-hétérocyclique aromatique et une source d'un anion autre qu'un halogénure.

2. Procédé suivant la revendication 1, caractérisé en ce que la polyoléfine possède un poids moléculaire moyen en nombre (Mₙ) qui varie de 200 à 100 000.

3. Procédé suivant la revendication 1 ou la revendication 2, caractérisé en ce que la polyoléfine est le polyisobutylène.

4. Procédé suivant l'une quelconque des revendications 1 à 3, caractérisé en ce que le dérivé d'amine primaire est un composé qui répond à la formule générale suivante : 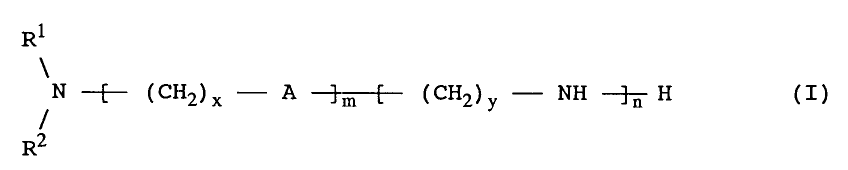 dans laquelle chaque symbole x a une valeur qui varie de 1 à 3, chaque symbole y a une valeur qui varie de 1 à 3, chaque symbole A représente indépendamment -O-ou -S-, m est 0, 1 ou 2 et n a une valeur qui varie de 1 à 10, et dans laquelle lorsque m est égal à 1, n est égal à 1 et A représente -O- ou -S-, ou n est 2 et A représente -S-, et lorsque m est égal à 2, n est égal à 1; et R¹ et R² ont les significations qui leur ont été attribuées dans la revendication 1.

5. Procédé suivant la revendication 4, caractérisé en ce que, dans la formule I, chacun des symboles R¹ et R² représente indépendamment un atome d'hydrogène ou le radical méthyle, y est égal à 2 ou à 3, m est égal à 0 et n est un nombre dont la valeur varie de 1 à 6.

6. Procédé suivant l'une quelconque des revendications précédentes, caractérisé en ce qu'on l'entreprend à une température qui varie de 160 à 170°C.

7. Procédé suivant l'une quelconque des revendications précédentes, caractérisé en ce qu'on l'effectue sous une une pression totale qui varie de 2 000 à 6 000 kPa.

8. Procédé suivant l'une quelconque des revendications précédentes, caractérisé en ce que la source de rhodium cationique ou de ruthénium cationique est un oxyde, un sel, un hydrure ou un carbonyle.

9. Procédé suivant l'une quelconque des revendications précédentes, caractérisé en ce que le ligand N-hétérocyclique aromatique est une 1,10-phénanthroline, une 2,2'-bipyridine, une quinoléine, ou une pyridine, éventuellement substituée.

10. Procédé suivant l'une quelconque des revendications précédentes, caractérisé en ce que l'anion est un anion nitrate, sulfate, sulfonate, borate, phosphate, phosphonate, carboxylate ou acétylacétonate.
